(19) Europäisches Patentamt

European Patent Office

Office européen des brevets

(11) **EP 1 722 311 A1**

(12) **EUROPEAN PATENT APPLICATION**

(43) Date of publication:
**15.11.2006 Bulletin 2006/46**

(51) Int Cl.:
***G06F 19/00*** (2006.01)

(21) Application number: **06009333.3**

(22) Date of filing: **05.05.2006**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(30) Priority: **09.05.2005 JP 2005136182**

(71) Applicant: **SYSMEX CORPORATION
Kobe-shi,
Hyogo 651-0073 (JP)**

(72) Inventors:
• **Kouchi, Yasuhiro
Chuo-ku
Kobe-shi
Hyogo 651-0073 (JP)**

• **Saitou, Takeo
Chuo-ku
Kobe-shi
Hyogo 651-0073 (JP)**
• **Seike, Masayoshi
Chuo-ku
Kobe-shi
Hyogo 651-0073 (JP)**
• **Nakajima, Hiromu
Chuo-ku
Kobe-shi
Hyogo 651-0073 (JP)**

(74) Representative: **HOFFMANN EITLE
Patent- und Rechtsanwälte
Arabellastrasse 4
81925 München (DE)**

(54) **Simulation system for functions of biological organs and recording medium in which program therefor is recorded**

(57) A simulation system for simulating functions of biological organs, comprising a biological model in which the functions of the biological organs are expressed by mathematical models. The biological model comprises a hepatic metabolism model block having specified input and output relating to hepatic function for simulating the hepatic function. The system further comprises arithmetic means for calculating an output value by using measurable status variables of a liver based on input value to the hepatic metabolism model block.

Fig. 2

**Description**

BACKGROUND OF THE INVENTION

The invention relates to a simulation system for simulating functions of biological organs, in particular, absorption, accumulation and metabolism functions of glucose by organs, and secretion, transportation and operation functions of insulin by organs by using a computer, and a recording medium in which program therefor is recorded.

To express a composition concentration in a body, in particular, blood glucose level and serum insulin concentration, it has been hitherto attempted to describe by using numerical models from the medical reason represented by diagnosis of diabetes mellitus.

Usable models include, for example, a minimal model by Bergman (see, for example, Bergman et al., American Journal of Physiology, Vol. 236, No. 6, pp. E-667-77, 1979, or Bergman et al., Journal of Clinical Investigation, Vol. 68, No. 6, pp. 1456-67, 1981).

This minimal model operates on variables, such as blood glucose level, plasma insulin concentration, and insulin action amount at insulin action point of peripheral tissue, that is, remote insulin. Supposing, at time t, the blood glucose level to be G (t), plasma insulin concentration to be I (t), and remote insulin to be X (t), the values of G (t), I (t), and X (t) are expressed in the following differential equations with the time differential values being taken on the left side.

$$dG(t)/dt=-p_1(G(t)-G_b)-X(t)G(t)$$

$$dX(t)/dt=-p_2X(t)+p_3(I(t)-I_b)$$

$$dI(t)/dt=-n(I(t)-I_b)+\gamma(G(t)-h) \quad (\text{provided } G(t) > h)$$
$$=-n(I(t)-I_b)+\gamma(G(t)-h) \quad (\text{provided } G(t) <= h)$$

In these equations, parameters are:

$p_1$:   insulin-independent glucose metabolism speed
$G_b$:   basal glucose level
$p_2$:   insulin uptake capacity at insulin action point
$p_3$:   insulin consumption rate to insulin-dependent glucose metabolism
$I_b$:   basal insulin concentration
n:   insulin consumption per unit time
$\gamma$:   insulin secretion sensitivity to glucose stimulation
h:   blood glucose threshold to start insulin secretion

These values are different in individual subjects.

In the human body, by nature, the blood glucose is controlled by mutual relation of four model blocks, that is, the pancreas for secreting insulin depending on stimulation of blood glucose, the liver for uptaking glucose from the blood and discharging glucose into the blood depending on the insulin concentration and blood glucose, the cardiovascular kinetic system for distributing insulin in peripheral tissues, and the peripheral tissues for receiving the action of insulin and metabolizing the glucose. On the other hand, in the above minimal model, constituent elements of models are abstract models not corresponding to four model blocks of the body, and it is hard to consider the simulation results of blood glucose variations and insulin concentration variations in the body in relation to four model blocks of the body.

Other reproducing techniques for blood glucose include prediction of blood glucose in diabetic patients (see, for example, Japanese Unexamined Patent Application No. 296598/1999).
According to this method, the blood glucose can be predicted, but the status of the organs relating to control of blood glucose cannot be known.

SUMMARY OF THE INVENTION

The invention is devised in the light of the above background, and it is hence an object thereof to present a simulation system capable of knowing the hepatic function status (pathology) useful for glycometabolism in the liver, especially treatment of diabetes mellitus, and a recording medium in which program therefor is recorded.

The simulation system of the invention is a simulation system for simulating functions of biological organs, comprising:

a biological model in which the functions of the biological organs are expressed by mathematical models,

wherein the biological model comprises a hepatic metabolism model block having specified input and output relating to hepatic function for simulating the hepatic function, and

wherein the system further comprises arithmetic means for calculating an output value by using measurable status variables of a liver based on input value to the hepatic metabolism model block.

The recording medium of the invention is a recording medium for recording a computer program which enables a computer to function as a simulation system of functions of biological organs,

wherein the computer program comprises a program code which operates the computer to function as a hepatic metabolism model block having specified input and output relating to hepatic function, in order to simulate functions of biological organs by using a biological model in which the functions of the biological organs are expressed by mathematical models, and

wherein the program code operates the computer to calculate the output value by using measurable status variables of a liver based on input value to the hepatic metabolism model block.

BRIEF DESCRIPTION OF THE DRAWINGS

Fig. 1 is a block diagram of hardware configuration of system according to one embodiment of the invention;
Fig. 2 is a block diagram of entire configuration of an example of biological model used in the system of the invention;
Fig. 3 is a block diagram of configuration of pancreas model in an example of biological model used in the system of the invention;
Fig. 4 is a block diagram of configuration of hepatic metabolism model in an example of biological model used in the system of the invention;
Fig. 5 is a block diagram of configuration of insulin kinetics model in an example of biological model used in the system of the invention;
Fig. 6 is a block diagram of configuration of peripheral metabolism model in an example of biological model used in the system of the invention;
Fig. 7 is a graph of glucose absorption speed used as input in an example of the invention;
Fig. 8 is a graph of blood glucose simulated in an example of the invention;
Fig. 9 is a graph of serum insulin concentration simulated in an example of the invention;
Fig. 10 is a graph of hepatic sugar uptake simulated in an example of the invention;
Fig. 11 is a graph of hepatic sugar release simulated in an example of the invention;
Fig. 12 is a graph of blood glucose for reference;
Fig. 13 is a graph of serum insulin concentration for reference; and

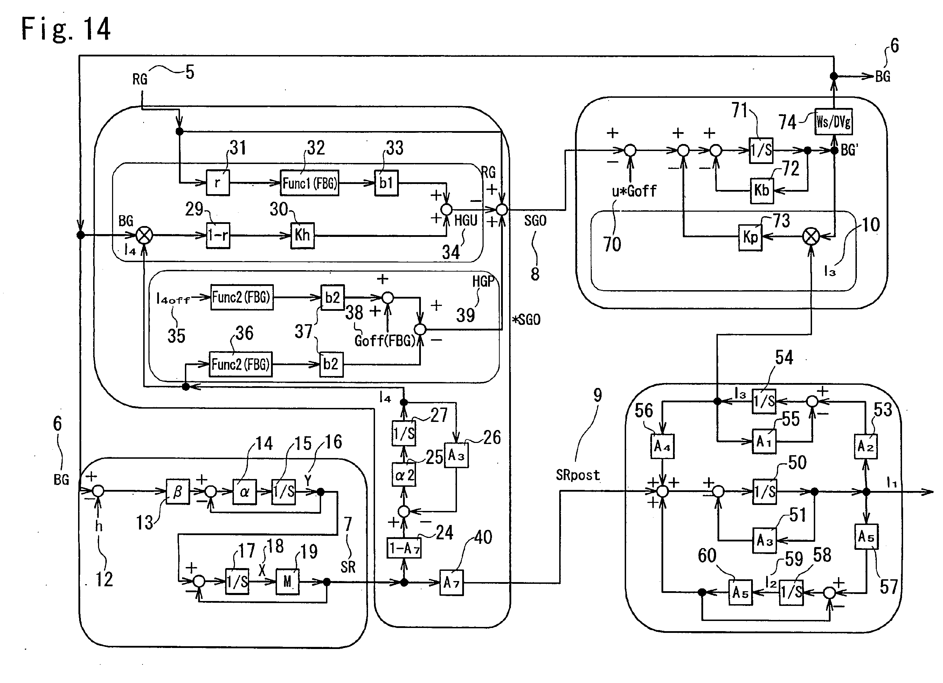

Fig. 14 is a diagram of an overall configuration of an example of biological model used in the invention.

DETAILED DESCRIPTION

Referring now to the accompanying drawings, embodiments of the simulation system (hereinafter, also referred to as "system") of the invention are specifically described below.

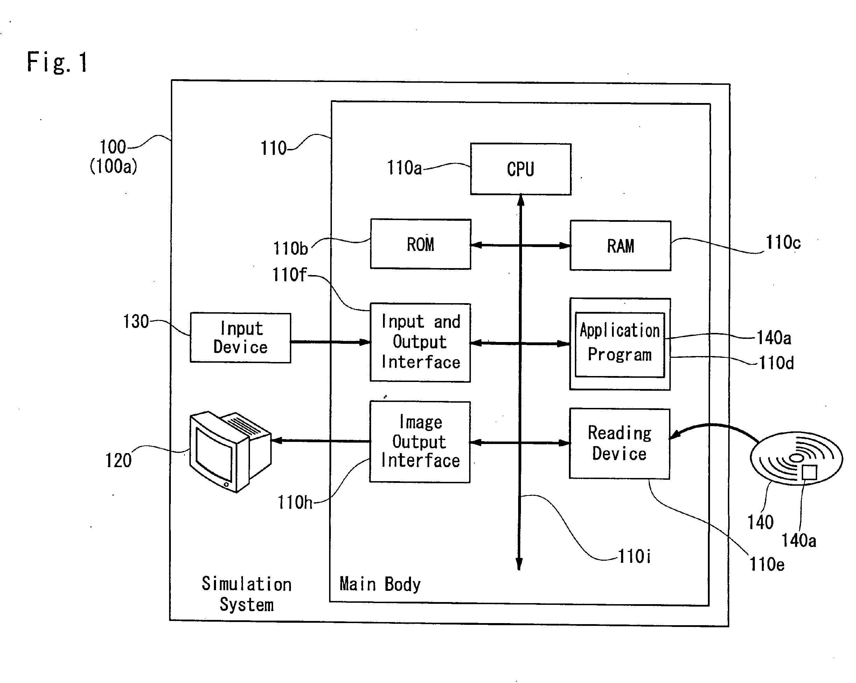

Fig. 1 is a block diagram of hardware configuration of system according to one embodiment of the invention. The system 100 in this embodiment includes a computer 110a mainly composed of a main body 110, a display 120, and an input device 130. The main body 110 is mainly composed of CPU 110a, ROM 110b, RAM 110c, hard disk 110d, reading device 110e, input and output interface 110f, communication interface 110g, and image output interface 110h, and the CPU 110a, ROM 110b, RAM 110c, hard disk 110d, reading device 110e, input and output interface 110f, and image output interface 110h are connected so as to exchange data with each other by means of bus 110i.

The CPU 110a can execute the computer program stored in the ROM 110b, and the computer program loaded in the RAM 110c. When an application program 140a described below is executed by the CPU 110a, the function blocks described below are realized, and the computer 100a functions as the system 100.

The ROM 110b comprises mask ROM, PROM, EPROM, EEPROM and the like, and stores the computer program to be executed by the CPU 110a and the data used therefor.

The RAM 110c comprises SRAM, DRAM and the like. The RAM 110c is used in reading of computer program stored in the ROM 110b and hard disk 110d. When executing these computer programs, it is also used as the working area of CPU 110a.

In the hard disk 110d, there are installed various computer programs to be executed by the CPU 110a such as operating system and application programs, and data to be used in execution of aforementioned computer programs. The application program 140a described below is also installed in this hard disk 110d.

The reading device 110e comprises flexible disk drive, CD-ROM drive, DVD-ROM drive and the like, and is capable of reading out the computer program or data recorded in a portable recording medium 140. The portable recording medium 140 stores the application program 140a for functioning the computer as the system of the invention, and the computer 100a can read out the application program 140a relating to the invention from the portable recording medium 140, to install the application program 140a in the hard disk 110d.

The aforementioned application program 140a can be presented not only by the portable recording medium 140, but also from an external device connected to the computer 100a to communicate with each other through an electric communication line (wired or wireless). For example, the aforementioned application program 140a is stored in the hard disk of server computer on the Internet, and the computer 100a accesses this server computer to download the aforementioned computer program, to install the computer program in the hard disk 110d.

In the hard disk 110d, the operating system for presenting graphical user interface environment such as Windows (registered trademark) of Microsoft Corporation of the United States is installed. In the following explanation, it is supposed that the application program 140a of the embodiment operates on this operating system.

The input and output interface 110f comprises, for example, Parallel interface such as USB, IEEE1394 or RS-232C, parallel interface such as SCSI, IDE or IEEE1284, and analog interface composed of D/A converter and A/D converter. An input device 130 having keyboard and mouse is connected to the input and output interface 110f, and the user handles the aforementioned input device 130, and can enter data in the computer 100a.

The image output interface 110h is connected to a display 120 comprising LCD, CRT and the like and outputs the video signal depending on the image data given from the CPU 110a to the display 120. The display 120 shows the image (screen) according to the given video signal.

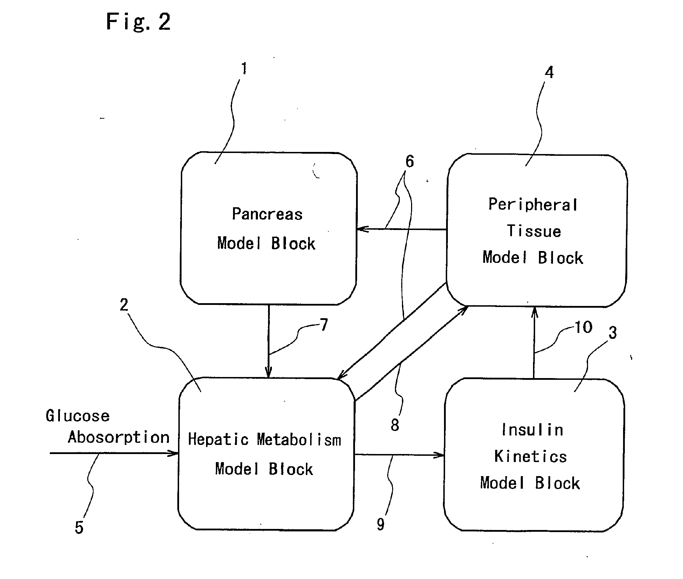

Fig. 2 is a block diagram of entire configuration of an example of biological model used in the system of the invention. As shown in Fig. 2, the biological model of the system of the invention is composed of a pancreas model block 1, a hepatic metabolism model block 2, an insulin kinetics model block 3, and a peripheral tissue model block 4, and each block has its own input and output. That is, the pancreas model block 1 receives a serum glucose concentration 6, and issues an insulin secretion speed 7. The hepatic metabolism model block 2 receives the glucose absorption 5 from digestive tract, serum glucose concentration 6, and insulin secretion speed 7, and issues the net glucose release 8 and posthepatic insulin 9. The insulin kinetics model block 3 receives the posthepatic insulin 9, and issues the insulin concentration 10 in peripheral tissues. The peripheral tissue model block 4 receives the net glucose release 8 and insulin concentration 10 in peripheral tissues, and issues the serum glucose concentration 6. The glucose absorption 5 is the data given from outside, and this function is realized, for example, by the user's input operation of inspection data by using the input device 130. These functional blocks 1 to 4 are realized by the computer program 140a executed by the CPU 110a.

In the system of the invention, since output values are issued by using status variables that can be measured by the hepatic metabolism model block for simulating the function of the liver, by comparing the simulation results with the actually measured status variables, parameters of mathematical model for expressing the hepatic functions can be optimized. As a result, a model expressing the hepatic functions closely is realized, and hepatic functions relating to pathology of the liver can be simulated accurately.

Together with the hepatic metabolism model block 2, the biological model includes the pancreas model block 1 expressing the pancreatic functions, insulin kinetics model block 3 expressing functions of insulin kinetics, and peripheral tissue model block 4 expressing the functions of peripheral tissues, and when the hepatic metabolism model block 2 is a model of three inputs and two outputs, receiving glucose from digestive tract, blood glucose, and insulin secretion from pancreas model block, and issuing net glucose release from liver and insulin concentration, it includes blocks corresponding to the pancreas, liver, insulin kinetics, and peripheral tissues relating to control of blood glucose as constituent elements. Thus, these functions are expressed in the unit system equivalent to the measured values, so that a simulation easy to understand the meaning thereof medically can be realized. At the same time, the entire function of biological organs relating to the liver can be simulated, and sugar metabolism in the liver, especially the pathological information about diabetes mellitus can be obtained comprehensively. A proper treatment can be given according to the obtained information.

The detail of each block mentioned above is discussed below. Herein, FGB and Ws respectively represent the fasting blood glucose (FGB = BG (0)) and the assumed body weight, and DVg and DVi are distribution volume to glucose and distribution volume to insulin.

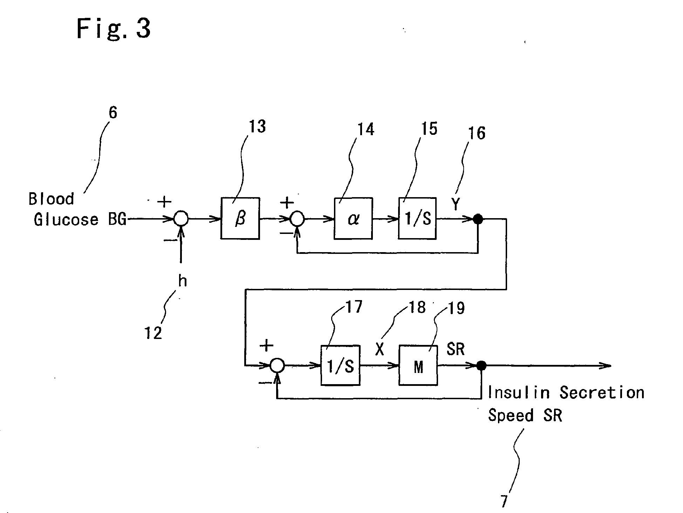

The input-output relation of pancreas model block 1 can be expressed in differential equation (1). It can be also expressed by using a block diagram expressed in Fig. 3, equivalent to differential equation (1).

```
Differential equation (1):

dY/dt=-α{Y(t)-β(BG(t)-h)}      (provided BG(t) > h)

     =-αY(t)                   (provided BG(t) <= h)

dX/dt=-M•X(t)+Y(t)

SR(t)=M•X(t)
```

Variables:

BG (t): blood glucose
X (t): total insulin that can be secreted from pancreas
Y (t): insulin supply speed newly supplied to glucose stimulation
SR (t): insulin secretion speed from pancreas

Parameters:

h: threshold of glucose concentration capable of stimulating insulin supply
α: following performance to glucose stimulation
β: sensitivity to glucose stimulation
M: secretion speed per unit concentration

In Fig. 2, the blood glucose 6, an input to the pancreas model block 1, corresponds to BG (t), and insulin secretion speed 7, an output, corresponds to SR (t).

In the block diagram in Fig. 3, reference numeral 6 is blood glucose BG (t), 7 is insulin secretion speed SR (t) from pancreas, 12 is threshold h of glucose concentration capable of stimulating insulin supply, 13 is susceptibility β to glucose stimulation, 14 is following performance α to glucose stimulation, 15 is integral element, 16 is insulin supply speed Y (t) newly supplied to glucose stimulation, 17 is integral element, 18 is total insulin X (t) that can be secreted from pancreas, and 19 is secretion speed M per unit concentration.

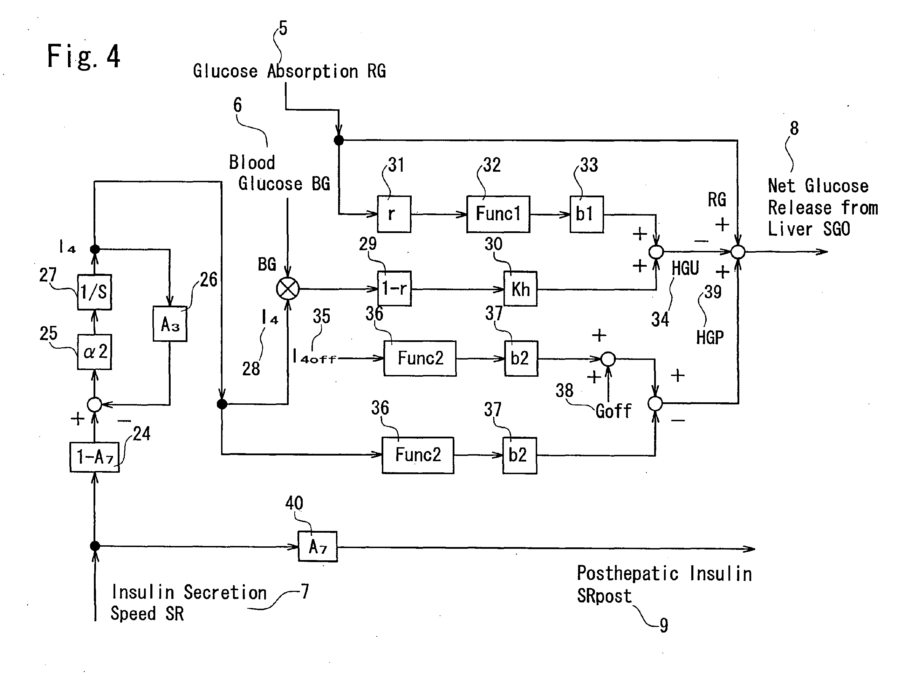

The input-output relation of hepatic metabolism model block 2 can be expressed in the following differential equation (2). It can be also expressed by using a block diagram expressed in Fig. 4, equivalent to differential equation (2).

Differential equation (2):

$$dI_4(t)/dt = \alpha2\{-A_3I_4(t) + (1-A_7) \cdot SR(t)\}$$

$$Goff(FGB) = f1 \qquad \text{(provided FGB < f3)}$$

$$= f1 + f2 \cdot (FGB-f3) \qquad \text{(provided FGB >= f3)}$$

$$Func1(FGB) = f4 - f5 \cdot (FGB - f6)$$

$$Func2(FGB) = f7/FGB$$

$$b1(I_4(t)) = f8\{1 + f9 \cdot I4(t)\}$$

$$HGU(t) = r \cdot Func1(FGB) \cdot b1(I_4(t)) \cdot RG(t) + (1-r) \cdot Kh \cdot BG(t) \cdot I_4(t)$$

$$\text{(provided HGU (t) >= 0)}$$

$$HGP(t) = I_{4off} \cdot Func2(FGB) \cdot b2 + G_{off}(FGB) - I_4(t) \cdot Func2(FGB) \cdot b2$$

$$\text{(provided HGP (t) >= 0)}$$

$$SGO(t) = RG(t) + HGP(t) - HGU(t)$$

$$SRpost(t) = A_7 SR(t)$$

Variables:

BG (t): blood glucose
SR (t): insulin secretion speed from pancreas
SRpost (t): posthepatic insulin
RG (t): glucose absorption from digestive tract
HGP (t): hepatic glucose release
HGU (t): hepatic glucose uptake
SGO (t): net glucose from liver
$I_4$ (t): liver insulin concentration

Parameters:

Kh: hepatic glucose uptake speed per unit insulin or per unit glucose
$A_7$: insulin uptake rate in liver
Goff: glucose release speed to basal metabolism
b2: adjustment term relating to hepatic glucose release suppression rate
r: insulin-independent hepatic glucose uptake distribution rate
$\alpha2$: propagation efficiency to hepatic insulin
$I_{4off}$: threshold of insulin concentration suppressing hepatic glucose release

Functions:

Goff (FGB): glucose release speed to basal metabolism
Func1 (FGB): hepatic glucose uptake rate to glucose stimulation from digestive tract

Func2 (FGB): hepatic glucose release suppression rate to insulin stimulation
f1 to f9: constants used in expression of three elements above
b1 ($I_4$ (t)): adjustment term relating to hepatic glucose uptake rate

Inputs to hepatic metabolism model block in Fig. 2, that is, glucose absorption 5 from digestive tract corresponds to RG (t), blood glucose 6 to BG (t), and insulin secretion speed 7 to SR (t), respectively. Outputs, net glucose release 8 corresponds to SGO (t), and posthepatic insulin 9 to SRpost (t), respectively.

In the block diagram in Fig. 4, reference numeral 5 is glucose absorption RG (t) from digestive tract, 6 is blood glucose BG (t), 7 is insulin secretion speed SR (t) from pancreas, 8 is net glucose SGO (t) from liver, 9 is posthepatic insulin SRpost (t), 24 is insulin passing rate (1-A7) in liver, 25 is propagation efficiency $\alpha$ 2 to hepatic insulin, 26 is posthepatic insulin distribution speed A3, 27 is integral element, 28 is hepatic insulin concentration $I_4$ (t), 9 is insulin-dependent hepatic glucose uptake distribution rate (1-r), 30 is hepatic glucose uptake speed Kh per unit insulin or unit glucose, 31 is insulin-independent hepatic glucose uptake distribution rate r, 32 is hepatic glucose uptake rate Func1 (FGB) to glucose stimulation from digestive tract, 33 is adjustment term b1 ($I_4$ (t)) relating to hepatic glucose uptake rate, 34 is hepatic glucose uptake HGU (t), 35 is threshold of insulin concentration $I_{4off}$ suppressing hepatic glucose release, 36 is hepatic glucose release suppression rate Func2 (FGB) to insulin stimulation, 37 is adjustment term b2 relating to hepatic glucose release suppression rate, 38 is glucose release speed to basal metabolism, 39 is hepatic glucose release HGP (t), and 40 is insulin uptake rate $A_7$ in liver, respectively.

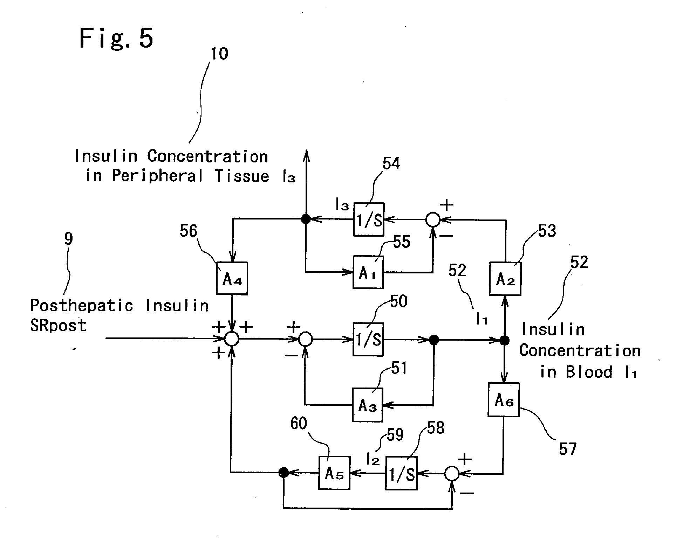

The input-output relation of insulin kinetics secretion can be expressed in the following differential equation (3). It can be also expressed by using a block diagram expressed in Fig. 5, equivalent to differential equation (3).

Differential equation (3):

$$dI_1(t)/dt = -A_3 I_1(t) + A_5 I_2(t) + A_4 I_3(t) + SRpost(t)$$

$$dI_2(t)/dt = A_6 I_1(t) - A_5 I_2(t)$$

$$dI_3(t)/dt = A_2 I_1(t) - A_1 I_3(t)$$

Variables:

SRpost (t): posthepatic insulin
$I_1$ (t) insulin concentration in blood
$I_2$ (t) : insulin concentration in insulin-independent tissues
$I_3$ (t): insulin concentration in peripheral tissues

Parameters:

$A_1$: insulin loss speed in peripheral tissues
$A_2$: insulin distribution rate to peripheral tissues
$A_3$: posthepatic insulin distribution speed
$A_4$: postperipheral insulin flow-out speed
$A_5$: insulin loss speed in insulin-independent tissues
$A_6$: insulin distribution rate in insulin-independent tissues
As the input to the insulin kinetics model block in Fig. 2, posthepatic insulin 9 corresponds to SRpost (t), and as the output, insulin concentration 10 in peripheral tissues corresponds to $I_3$ (t).

In the block diagram in Fig. 5, reference numeral 9 is posthepatic insulin SRpost (t), 10 is insulin concentration $I_3$ (t) in peripheral tissues, 50 is integral element, 51 is posthepatic insulin distribution speed $A_3$, 52 is insulin concentration in blood $I_1$ (t), 53 is insulin distribution rate $A_2$ to peripheral tissues, 54 is integral element, 55 is insulin loss speed A1 in peripheral tissues, 56 is postperipheral insulin flow-out speed $A_4$, 57 is insulin distribution rate $A_6$ to insulin-independent tissues, 58 is integral element, 59 is insulin concentration $I_2$ (t) in insulin-independent tissues, and 60 is insulin loss speed $A_5$ in insulin-independent tissues, respectively.

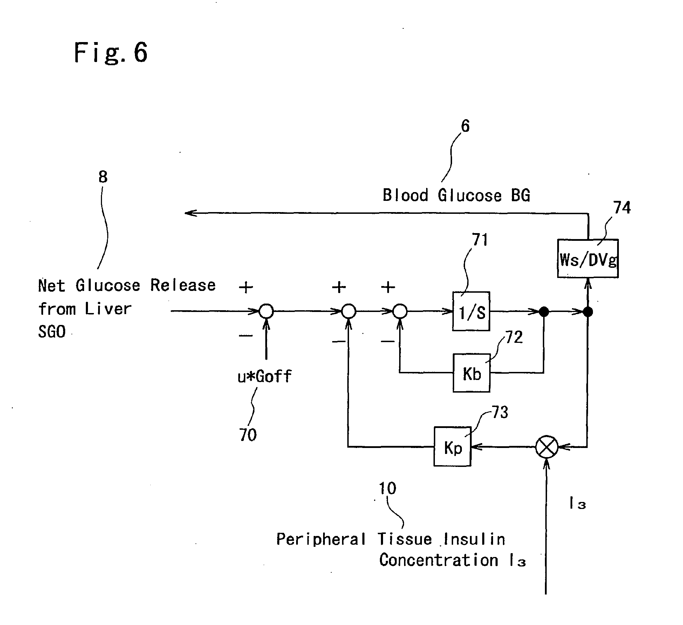

The input-output relation of peripheral tissue model block 4 can be expressed in the following differential equation (4). It can be also expressed by using a block diagram expressed in Fig. 6, equivalent to differential equation (4).

```
Differential equation (4):

dBG'/dt=SGO(t)-u*Goff(FGB)-Kb•BG'(t)-Kp•I₃(t)•BG'(t)
```

Variables:

BG' (t): blood glucose (provided BG [mg/dl], BG' [mg/kg])
SGO (t): net glucose from liver
$I_3$ (t): insulin concentration in peripheral tissues

Parameters:

Kb: insulin-independent glucose consumption speed in peripheral tissues
Kp: insulin-dependent glucose consumption speed in peripheral tissues per unit insulin or unit glucose
u: rate occupied by insulin-independent glucose consumption to basal metabolism, in glucose release speed to basal metabolism Functions:
Goff (FGB): glucose release speed to basal metabolism
f1 to f3: constants used in expression of Goff

As the inputs to the peripheral tissue model block in Fig. 2, insulin concentration in peripheral tissues 10 corresponds to $I_3$ (t), and net glucose from liver 8 to SGO (t), and as the output, blood glucose 6 corresponds to BG (t).
In the block diagram in Fig. 6, reference numeral 6 is blood glucose BG (t), 8 is net glucose SGO (t) from liver, 10 is insulin concentration $I_3$ (t) in peripheral tissues, 70 is insulin-independent glucose consumption speed to basal metabolism u*Goff (FGB), 71 is integral element, 72 is insulin-independent glucose consumption speed Kb in peripheral tissues, 73 is insulin-dependent glucose consumption speed Kp in peripheral tissues per unit insulin or unit glucose, and 74 is unit conversion constant Ws/DVg.
As shown in Fig. 2, the input and output between blocks for constituting the system are mutually connected, and by applying the glucose absorption 5 from the digestive tract, the time-series changes of blood glucose and insulin concentration can be calculated according to the formula and simulated.
The sequentially calculated blood glucose and insulin concentration can be shown in the display 120. As a result, the user can easily recognize the result simulating the biological organs. This system can be employed as sub-system for simulating the biological functions in the medical system such as diagnostic support system of diabetes mellitus. In this case, the calculated time-series changes of blood glucose and insulin concentration can be transferred to other constituent elements of the medical support system, and medical information of high reliability such as diagnosis support information for diabetes mellitus can be obtained on the basis of the blood glucose and insulin concentration calculated by this system.
For calculation of differential equation in this system, for example, E-Cell (the software laid open by Keio University) or MatLab (product of The MathWorks, Inc.) may be used, but other computation systems may be alternatively used.
The following is the simulated example of time-series changes of blood glucose, insulin concentration, hepatic glucose uptake and hepatic glucose release measured by using the system of the invention. In this example, values shown in Table 1 are used as parameters of blocks.

Table 1

|  | Parameter | Value | Unit |
|---|---|---|---|
| Pancreas | h | 92.43 | [mg/dl] |
|  | α | 0.228 | [1/min] |
|  | β | 0.357 | [(μU/ml) · (dl/mg) · (1/min)] |
|  | M | 1 | [1/min] |

(continued)

|  | Parameter | Value | Unit |
|---|---|---|---|
| Insulin Kinetics | $A_1$ | 0.025 | [1/min] |
|  | $A_2$ | 0.042 | [1/min] |
|  | $A_3$ | 0.435 | [1/min] |
|  | $A_4$ | 0.02 | [1/min] |
|  | $A_5$ | 0.394 | [1/min] |
|  | $A_6$ | 0.142 | [1/min] |
| Peripheral Tissues | Kb | 0.009 | [1/min] |
|  | Kp | 5.28E-05 | [(ml/$\mu$U) · (1/min) · (dl/kg)] |
|  | u | 0.6 |  |
| Liver | $A_7$ | 0.47 |  |
|  | Kh | 0. 0000462 | [(ml/$\mu$U) · (1/min) · (dl/kg)] |
|  | b2 | 1.1 |  |
|  | r | 0.98 |  |
|  | $\alpha2$ | 0.228 |  |
|  | $I_{4off}$ | 5 | [$\mu$U/ml] |

For calculating the differential equations, values in Table 2 are used as examples of initial values of variables.

Table 2

|  | Initial Value | Value | Unit |
|---|---|---|---|
| Pancreas | X(0) | 336.4 | [$\mu$U/ml] |
|  | Y(0) | 4.4 | [($\mu$U/ml) · (1/min)] |
| Insulin Kinetics | $I_1(0)$ | 8 | [$\mu$U/ml] |
|  | $I_2(0)$ | $I_1(0)*(A_2/A_1)$ | [$\mu$U/ml] |
|  | $I_3(0)$ | $I_1(0)*(A_6/A_5)$ | [$\mu$U/ml] |
|  | $I_4(0)$ | 4 | [$\mu$U/ml] |
| Liver | FBG | 115 | [mg/dl] |

As examples of constants and reference values used in these examples, the values in Table 3 are used.

Table 3

| | Constant | Value | Unit |
|---|---|---|---|
| Function | f1 | 1. 8 | [(mg/kg) · (1/m in) ] |
| | f2 | 0.02 | [(dl/kg) · (1/min)] |
| | f3 | 140 | [mg/dl] |
| | f4 | 0. 525 | |
| | f5 | 0. 005 | [(dl/mg)] |
| | f6 | 80 | [mg/dl] |
| | f7 | 13.2 | [(ml/$\mu$U) · (mg/dl) · (mg/kg) · (1/min)] |
| | f8 | 0.9 | |
| | f9 | 0. 0001 | |
| Reference | Ws | 70 | [kg] |
| | DVg | 154 | [dl] |
| | DVi | 3.52 | [l] |

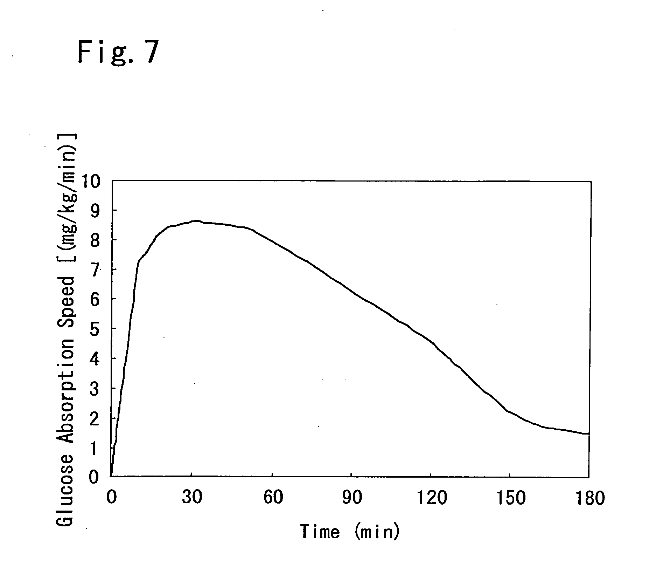

As the glucose absorption speed from digestive tract, the value shown in Fig. 7 is used.

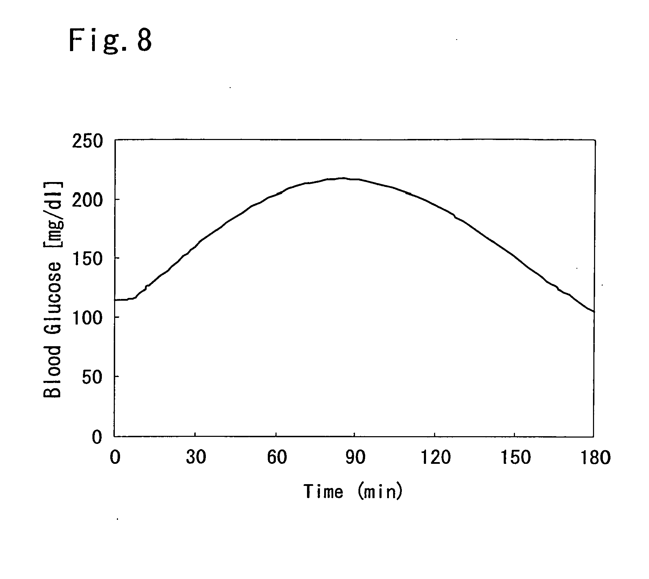

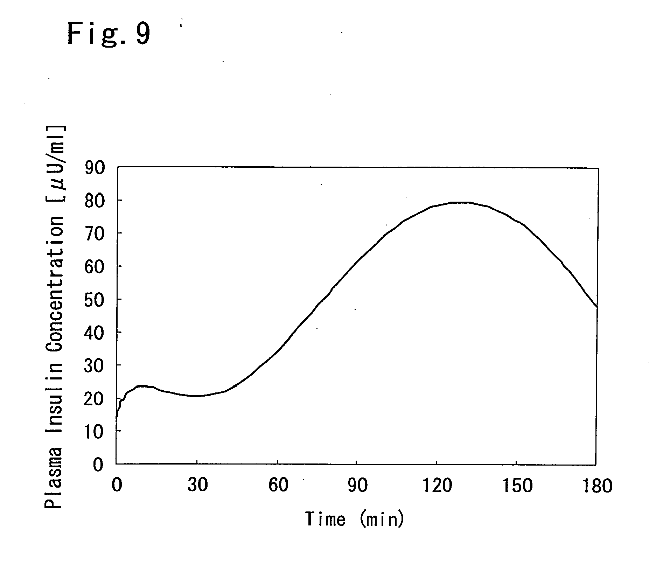

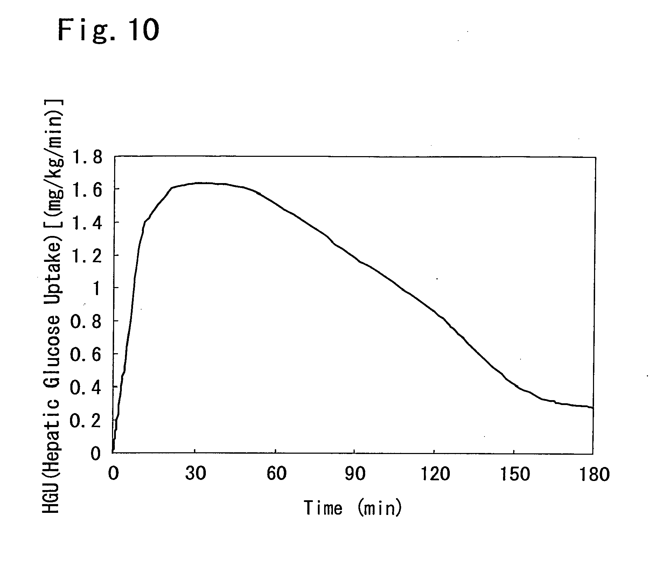

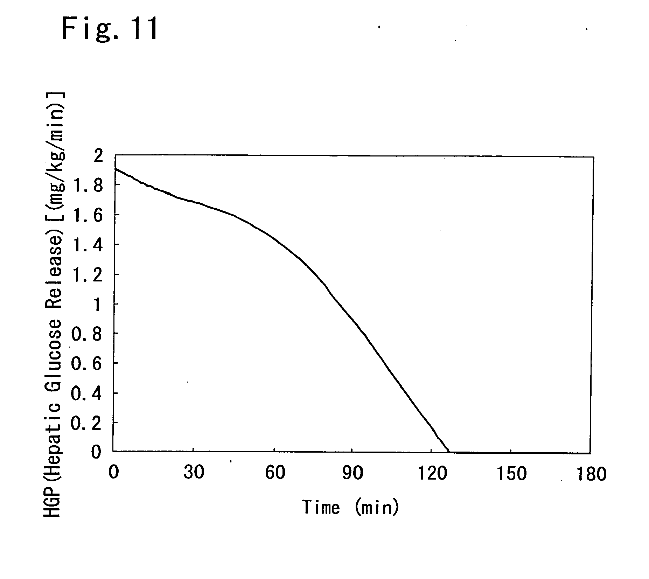

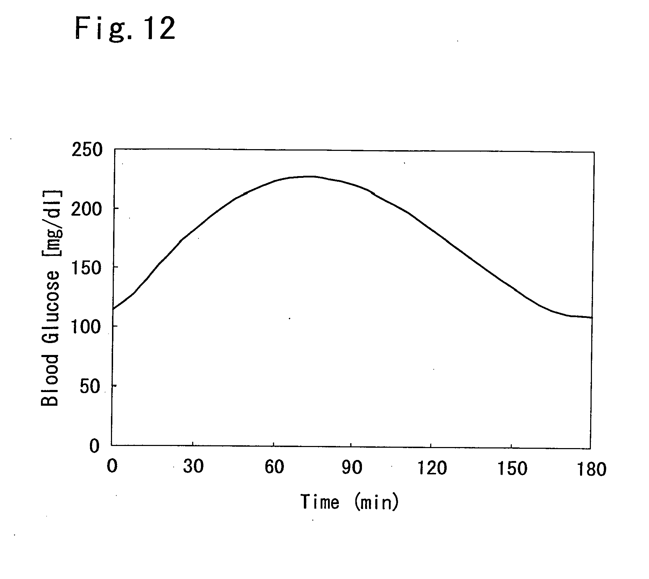

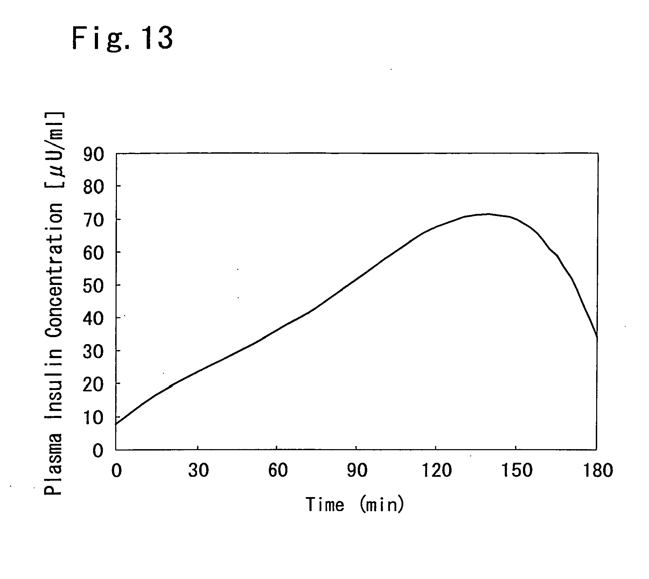

Among the simulation results in the above condition, the time-series variations of blood glucose 6 are shown in Fig. 8, time-series variations of serum insulin concentration 52 are shown in Fig. 9, time-series variations of hepatic glucose uptake are shown in Fig. 10, and time-series variations of hepatic glucose release are shown in Fig. 11. The blood glucose for reference is given in Fig. 12, and the serum insulin concentration for reference is given in Fig. 13.

Thus, by using this system, the time-series variations of blood glucose due to glucose absorption, insulin concentration, hepatic glucose uptake, and hepatic glucose release can be reproduced in variations similar to physiological variations. The models used in this system include blocks corresponding to pancreas, liver, insulin kinetics and peripheral tissues relating to control of blood glucose as constituent elements, and hence the meaning is easy to understand from the medical aspect.

The simulation system of the invention uses mathematical models describing the biological functions, and hence can reproduce time-series variations of blood glucose in the body, insulin concentration, hepatic glucose uptake and hepatic glucose release. If measurable status variations of the liver include hepatic glucose uptake and hepatic glucose release in hepatic model block, it is possible to obtain hepatic glucose uptake and hepatic glucose release by simulation which are important hepatic functions closely related to the diabetes mellitus, so that the information relating to pathology of diabetes mellitus can be obtained easily. On the basis of the information relating to the pathology, an appropriate treatment can be given. Besides, since mathematical models include blocks corresponding to pancreas, liver, insulin kinetics and peripheral tissues relating to control of blood glucose as constituent elements, it is useful for simulation of which meaning is easy to understand from the medical aspect.

**Claims**

1.   A simulation system for simulating functions of biological organs, comprising:

   a biological model in which the functions of the biological organs are expressed by mathematical models,

   wherein the biological model comprises a hepatic metabolism model block having specified input and output relating to hepatic function for simulating the hepatic function, and
   wherein the system comprises arithmetic means for calculating an output value by using measurable status variables of the liver on the basis of an input value to the hepatic metabolism model block.

2.   The simulation system of claim 1, wherein the biological model expresses the functions of glucose absorption, accumulation and metabolism.

3.   The simulation system of claim 1 or 2, wherein the biological model expresses the functions of insulin secretion, transportation, and action.

4. The simulation system of any one of claims 1 to 3, wherein the status variables comprise the hepatic glucose uptake and hepatic glucose release in the hepatic metabolism model block.

5. The simulation system of any one of claims 1 to 4, wherein the biological model comprises at least one of pancreas model block expressing the pancreatic functions, insulin kinetics model block expressing functions of insulin kinetics, and peripheral tissue model block expressing the functions of peripheral tissues.

6. The simulation system of any one of claims 1 to 4, wherein the biological model block further comprises pancreas model block expressing the pancreatic functions, insulin kinetics model block expressing functions of insulin kinetics, and peripheral tissue model block expressing the functions of peripheral tissues, and the hepatic metabolism model block is a model of three inputs and two outputs, receiving glucose from digestive tract, blood glucose, and insulin secretion from pancreas model block, and issuing net glucose release from liver and insulin concentration.

7. A recording medium for recording a computer program which enables a computer to function as a simulation system of functions of biological organs,
wherein the computer program comprises a program code which operates the computer to function as a hepatic metabolism model block having specified input and output relating to hepatic function, in order to simulate functions of biological organs by using a biological model in which the functions of the biological organs are expressed by mathematical models, and
wherein the program code operates the computer to calculate the output value by using measurable status variables of a liver based on input value to the hepatic metabolism model block.

8. The recording medium of claim 7, wherein the biological model expresses the functions of glucose absorption, accumulation and metabolism.

9. The recording medium of claim 7 or 8, wherein the biological model expresses the functions of insulin secretion, transportation, and action.

10. The recording medium of any one of claims 7 to 9, wherein the status variables comprise the hepatic glucose uptake and hepatic glucose release in the hepatic metabolism model block.

11. The recording medium of any one of claims 7 to 10, wherein the biological model comprises at least one of pancreas model block expressing the pancreatic functions, insulin kinetics model block expressing functions of insulin kinetics, and peripheral tissue model block expressing the functions of peripheral tissues.

12. The recording medium of any one of claims 7 to 10, wherein the biological model block further comprises pancreas model block expressing the pancreatic functions, insulin kinetics model block expressing functions of insulin kinetics, and peripheral tissue model block expressing the functions of peripheral tissues, and the hepatic metabolism model block is a model of three inputs and two outputs, receiving glucose from digestive tract, blood glucose, and insulin secretion from pancreas model block, and issuing net glucose release from liver and insulin concentration.

EP 1 722 311 A1

Fig. 1

Fig. 2

# Fig. 3

Fig. 4

Net Glucose Release from Liver SGO

8

Glucose Absorption RG

5

Blood Glucose BG

6

Posthepatic Insulin SRpost

9

Insulin Secretion Speed SR

7

RG

HGU

34

HGP

39

b1  33

Func1  32

Kh  30

b2  37

b2  37

r  31

1-r  29

Func2  36

Func2  36

Goff  38

BG

I₄  28

I₄off  35

A₃  26

1/S  27

α2  25

1-A₇  24

A₇  40

15

# Fig. 5

10

Insulin Concentration
in Peripheral Tissue $I_3$

9

Posthepatic Insulin
SRpost

54

$I_3$  56  55  53  52

$\boxed{1/S}$  $\boxed{A_1}$  52  $\boxed{A_2}$

$\boxed{A_4}$  $I_1$

$\boxed{1/S}$  50  Insulin
Concentration
in Blood $I_1$

$\boxed{A_3}$  51

60  59  58

$\boxed{A_5}$  $I_2$  $\boxed{1/S}$  $\boxed{A_6}$  57

# Fig. 6

Blood Glucose BG 6

74

8

Net Glucose Release from Liver SGO

71

1/S

Ws/DVg

u*Goff

70

Kb 72

Kp 73

10

Peripheral Tissue Insulin Concentration $I_3$

$I_3$

# Fig. 7

Fig. 8

Fig. 9

Fig. 10

Fig. 11

Fig. 12

# Fig. 13

Fig. 14

EP 1 722 311 A1

**European Patent Office**

**EUROPEAN SEARCH REPORT**

Application Number

EP 06 00 9333

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SANO A, NAITO Y, OHNO H, NAKAJIMA H & TOMITA M: "A Simulation Model of Cardiovascular System for Diabetes Pathophysiological Model Using E-Cell System" PROCEEDINGS OF THE INTERNATIONAL CONFERENCE ON GENOME INFORMATICS, UNIVERSAL ACADEMY PRESS,TOKYO, 2002, vol. 13, 16 December 2002 (2002-12-16), - 18 December 2002 (2002-12-18) pages 480-481, XP002389543 * the whole document * | 1-12 | INV. G06F19/00 |
| X | WO 02/087506 A (ENTELOS, INC; BRAZHNIK, PAUL; HALL, KEVIN; POLIDORI, DAVE; SILER, SCOT) 7 November 2002 (2002-11-07) * paragraph [0034] - paragraph [0046]; figure 3 * * paragraph [0084] - paragraph [0112] * * Appendix A: A1-A39 * | 1-12 | |
| X | US 2005/071141 A1 (BUTLER RICHARD) 31 March 2005 (2005-03-31) * paragraph [0065] - paragraph [0138]; figures 2,5,6 * | 1-12 | **TECHNICAL FIELDS SEARCHED (IPC)**<br>G06F |

-/--

The present search report has been drawn up for all claims

| Place of search | Date of completion of the search | Examiner |
|---|---|---|
| Munich | 10 July 2006 | Sanandrés Ledesma, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

**European Patent Office**

## EUROPEAN SEARCH REPORT

Application Number

EP 06 00 9333

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ERZEN F C ET AL: "Glucosim: a simulator for education on the dynamics of diabetes mellitus" PROCEEDINGS OF THE 23RD. ANNUAL INTERNATIONAL CONFERENCE OF THE IEEE ENGINEERING IN MEDICINE AND BIOLOGY SOCIETY. 2001 CONFERENCE PROCEEDINGS. (EMBS). INSTANBUL, TURKEY, OCT. 25 - 28, 2001, ANNUAL INTERNATIONAL CONFERENCE OF THE IEEE ENGINEERING IN M, vol. VOL. 1 OF 4. CONF. 23, 25 October 2001 (2001-10-25), pages 3163-3166, XP010593771 ISBN: 0-7803-7211-5 * page 3163, left-hand column, line 1 - page 3164, right-hand column, line 14 * * page 3165, right-hand column, line 8 - page 3166, left-hand column, line 12 * ----- | 1-12 | |
| P,X | EP 1 598 766 A (SYSMEX CORPORATION) 23 November 2005 (2005-11-23) * the whole document * ----- | 1-12 | TECHNICAL FIELDS SEARCHED (IPC) |

The present search report has been drawn up for all claims

| Place of search | Date of completion of the search | Examiner |
|---|---|---|
| Munich | 10 July 2006 | Sanandrés Ledesma, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 06 00 9333

This annex lists the patent family members relating to the patent documents cited in the above-mentioned European search report.
The members are as contained in the European Patent Office EDP file on
The European Patent Office is in no way liable for these particulars which are merely given for the purpose of information.

10-07-2006

| Patent document cited in search report | | Publication date | Patent family member(s) | | Publication date |
|---|---|---|---|---|---|
| WO 02087506 | A | 07-11-2002 | CA | 2445598 A1 | 07-11-2002 |
| | | | EP | 1389998 A2 | 25-02-2004 |
| | | | JP | 2005508025 T | 24-03-2005 |
| | | | US | 2003058245 A1 | 27-03-2003 |
| US 2005071141 | A1 | 31-03-2005 | CA | 2459673 A1 | 20-03-2003 |
| | | | EP | 1428165 A2 | 16-06-2004 |
| | | | WO | 03023682 A2 | 20-03-2003 |
| | | | GB | 2381907 A | 14-05-2003 |
| | | | JP | 2005502406 T | 27-01-2005 |
| EP 1598766 | A | 23-11-2005 | CN | 1696931 A | 16-11-2005 |
| | | | US | 2005256690 A1 | 17-11-2005 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11296598 A

**Non-patent literature cited in the description**

- **BERGMAN et al.** *American Journal of Physiology,* 1979, vol. 236 (6), E-667-77
- **BERGMAN et al.** *Journal of Clinical Investigation,* 1981, vol. 68 (6), 1456-67